# EUROPÄISCHE PATENTANMELDUNG

(11) **EP 2 223 616 A1**
(43) Veröffentlichungstag der Anmeldung: **01.09.2010**
(21) Anmeldenummer: 09152758.0
(22) Anmeldetag: 13.02.2009
(51) Int. Cl.: A23L 1/29, A23L 1/30, A61K 36/9066, A61K 36/82, A61K 36/48, A61K 36/752

(54) **Nahrungsergänzungsmittelpräparat mit einer Wirksubstanzmischung**

(71) Anmelder: PM-International AG, 1618 Luxembourg (LU)
(72) Erfinder: Schmitt, Gerhard Dr., 64625 Bensheim (DE); Fritzmeier, Franz Dr., 91710 Gunzenhausen (DE)
(74) Vertreter: Zech, Stefan Markus

(57) **Zusammenfassung**

Es wird ein Nahrungsergänzungsmittelpräparat mit einer Wirksubstanzmischung vorgeschlagen. Dabei enthält die Wirksubstanzmischung
- Trigonella foenum-graecum L. (Bockshornkleesamen),
- Camelia sinsensis mit einem Anteil von Polyphenolen und Coffein,
- Curcumaextrakt,
- Ayurvedisches Gewürzextrakt, insbesondere Pfefferextrakt sowie
- Bitterorangenextrakt.
Weiterhin ein Lebensmittelset umfassend:
- eine vorbestimmte Anzahl von Kapseln mit dem Nahrungsmittelpräparat,
- ein Begleitpräparat zur Entwässerung, Entsäuerung und Öffnung der Fettspeicher, enthaltend Mineralstoffe und Spurenelemente sowie mindestens einen Katalysator und/oder Wirkstoff, sowie
- einen Mahlzeitenersatz in Form eines oder mehrerer Shakes.

## Beschreibung

Die Erfindung betrifft ein Nahrungsergänzungsmittel mit einer Wirksubstanzmischung nach den Merkmalen des Patentanspruches 1 sowie ein diätisches Lebensmittelset nach den Merkmalen des Patentanspruches 11.

Bereits aus der EP 1 588 659 ist ein diätisches Lebensmittel bekannt, um Übergewicht entgegenzuwirken bzw. durch Einnahme über einen bestimmten Zeitraum Übergewicht zu reduzieren.

Die Aufgabe der vorliegenden Erfindung besteht darin, ein Nahrungsergänzungsmittelpräparat zu schaffen, das den Ablauf der vorbekannten Diät unterstützt bzw. eine Gewichtskontrolle bzw. Gewichtsreduzierung in noch effizienterem Maße ermöglicht.

Diese Aufgabe wird einerseits durch ein Nahrungsmittelergänzungspräparat nach den Merkmalen des Anspruches 1 sowie andererseits durch ein diätisches Lebensmittelset nach den Merkmalen des Anspruches 11 gelöst. Vorteilhafte Weiterbildungen sind in den Unteransprüchen angegeben.

Das erfindungsgemäß vorgeschlagene Nahrungsergänzungsmittelpräparat zeichnet sich durch eine Wirksubstanzmischung aus, die enthält:
- Trigonella foenum-graecum L. (Bockshornkleesamen),
- Camelia sinsensis mit einem Anteil von Polyphenolen und Coffein,
- Curcumaextrakt,
- Ayurvedisches Gewürzextrakt, insbesondere Pfefferextrakt sowie
- Bitterorangenextrakt.

Die verschiedenen Bestandteile wirken hierbei in synergetischer Weise zusammen.

Trigonella foenum-graecum L. (Bockshornkleesamen) kann dabei selbst verschiedene Wirkmechanismen hervorrufen, nämlich zunächst im Magen aufquellen und dadurch allein schon ein Gefühl der Sättigung erzeugen. Der Wirkmechanismus zwischen Magen und Gehirn beruht dabei auf entsprechenden nervalen Verbindungen und erfolgt über das vegetative Nervensystem. Zusätzlich kann foenum-graecum L. (Bockshornkleesamen) eine humorale Einwirkung auf das Hungerempfinden, insbesondere eine Dämpfung des Hungerempfindens auslösen. Schließlich kann foenum-graecum L. (Bockshornkleesamen) Zucker im Magen und Darm binden und so dessen Resorption verzögern, so dass der Zuckerspiegel nicht steil ansteigt und insofern weniger Insulin ausgeschüttet wird. Die Reduktion des Insulingehalts im Blut bewirkt wiederum eine bessere Fettverbrennung. Bekanntermaßen initiiert die Lipase die Fettverbrennung, wobei Insulin die Lipase bzw. Blutlipase blockiert.

Camelia Sinsensis ist beigefügt vorrangig wegen des hohen Anteils an Polyphenolen sowie an Coffein. Die Polyphenole unterstützen die Thermogenese sowie die Fettverbrennung. Das Coffein kann als Initiator zur Fettverbrennung angesehen werden. Es unterstützt die Energiebereitstellung und damit ebenfalls den Fettabbau.

Die Beimischung von Curcumaextrakt kann eine Appetitzügelung bewirken. Die Beimengung des ayurvedischen Gewürzextrakts, insbesondere Pfefferextrakts, stärkt die Resorption der Wirksubstanzmischung generell und unterstützt ebenfalls den Fettabbau.

Bitterorangenextrakt kann ebenfalls zur Gewichts- bzw. Körperfettreduktion beitragen, da es positiv auf die Thermogenese einwirkt.

In einer bevorzugten Ausführung ist Trigonella foenum-graecum L. in einem Anteil von 15 Gew.% bis 90 Gew.%, vorzugsweise in einem Anteil von 65 Gew.% bis 80 Gew.%, in der Wirksubstanzmischung enthalten.

Camelia Sinsensis kann vorzugsweise mit einem Anteil von 5 Gew.% bis 50 Gew.%, vorzugsweise mit einem Anteil von 20 Gew.% bis 30 Gew.% in der Wirksubstanzmischung enthalten sein.

Curcumaextrakt kann in einer bevorzugten Ausgestaltung mit einem Anteil von 0,2 Gew.% bis 2,5 Gew.%, vorzugsweise mit einem Anteil von 0,7 Gew.% bis 1,3 Gew.% in der Wirksubstanzmischung enthalten sein.

Weiter kann der ayurvedische Gewürzextrakt, insbesondere der Pfefferextrakt, mit einem Anteil von 0,05 Gew.% bis 0,8 Gew.%, vorzugsweise mit einem Anteil von 0,15 Gew.% bis 0,4 Gew.%, in der Wirksubstanzmischung enthalten sein.

In einer weiter bevorzugten Ausgestaltung kann Bitterorangenextrakt (Citrus aurantium) mit einem Anteil von 0,2 Gew.% bis 4,0 Gew.%, vorzugsweise mit einem Anteil von 0,6 Gew.% bis 2,0 Gew.%, in der Wirksubstanzmischung enthalten sein.

In einer weiter bevorzugten Ausgestaltung ist das Nahrungsergänzungsmittelpräparat insbesondere durch Bereitstellung in Kapseln so konfektioniert, dass die Tagesdosis für Trigonella foenum-graecum L. im Bereich von 300 mg bis 5 g, vorzugsweise im Bereich von 900 mg bis 1500 mg, und/oder die Tagesdosis von Camelia sinsensis im Bereich von 200 mg bis 2500 mg, vorzugsweise von 600 mg bis 1200 mg, und/oder die Tagesdosis von Curcumaextrakt im Bereich von 2 mg bis 100 mg, vorzugsweise im Bereich von 5 mg bis 30 mg, und/oder die Tagesdosis des ayurvedischen Gewürzextrakts, insbesondere des Pfefferextrakts, im Bereich von 1 mg bis 20 mg, vorzugsweise im Bereich von 2 mg bis 6 mg, und/oder die Tagesdosis des Bitterorangenextrakts im Bereich von 5 mg bis 50 mg, vorzugsweise im Bereich von 10 mg bis 30 mg, liegt.

Bevorzugtermaßen liegt der in Camelia Sinsensis enthaltene Anteil an Polyphenolen im Bereich von 5 Gew.% bis 20 Gew.%, vorzugsweise im Bereich von 8 Gew.% bis 15 Gew.%, bezogen auf die gesamte Wirksubstanzmischung. Die Tagesdosis an Polyphenolen liegt bevorzugterweise im Bereich von 75 mg bis 500 mg, vorzugsweise bei 150 mg bis 300 mg.

In einer weiter bevorzugten Ausgestaltung liegt der in Camelia Sinsensis enthaltene Anteil an Coffein im Bereich von 0,5 Gew.% bis 5 Gew.%, vorzugsweise im Bereich von 1,0 Gew.% bis 2,9 Gew.%, bezogen auf die Wirksubstanz. Die Tagedosierung von Coffein liegt bevorzugterweise im Bereich von 10 mg bis 100 mg, vorzugsweise in einem Bereich von 20 mg bis 50 mg.

In einer besonders bevorzugten Ausgestaltung ist die Wirksubstanz gegebenenfalls unter Beimengung von weiteren Träger- und Zusatzstoffen, insbesondere unter Beimengung von MCT-Öl, in oral einnehmbaren Kapseln konfektioniert. Das MCT-Öl wirkt in der gesamten Wirksubstanzmischung synergetisch hinsichtlich der Stoffwechselaktivierung, insbesondere günstig auf den Fettstoffwechsel.

Weiter wird ein diätisches Lebensmittel, umfassend eine vorbestimmte Anzahl von Kapseln mit dem Nahrungsergänzungsmittelpräparat nach einem der Ansprüche 1 bis 10, einem Begleitpräparat zur Entwässerung, Entsäuerung und Öffnung der Fettspeicher sowie einem Mahlzeitenersatz in Form eines oder mehrerer Shakes beansprucht. Das Begleitpräparat zur Entwässerung, Entsäuerung und Öffnung der Fettspeicher kann vorzugsweise die Mineralstoffe und SpurenelementeKalzium, Magnesium, Selen, Kupfer, Zink, Chrom und weiterhin Vitamin D, enthalten und weiterhin mindestens einen Katalysator und/oder Wirkstoff, vorzugsweise in Pulverform enthalten, der die Resorption der Mineralstoffe und/oder Spurenelemente steigert und/oder verbessert und/oder beschleunigt.

Als Begleitpräparat zur Entwässerung, Entsäuerung und Öffnung der Fettspeicher kann insbesondere ein Basenpulver, beispielsweise das Produkt Restorate®, in Betracht kommen. Es wird in diesem Zusammenhang auch auf die EP 1 719 519 B1 sowie die EP 1 698 344 B1, beide im Namen der hiesigen Anmelderin, Bezug genommen. Das Basenpulver unterstützt durch die Entsäuerung den Abbau des Fettübergewichts, da es die Fettzellen aufschließt, und fördert gleichzeitig über die Entsäuerung den Abbau des Wasser-Schlackenübergewichts. Eine Gewichtsabnahme durch Entsäuerung findet relativ schnell statt und kann bei "verschlackten" Personen über Wasser-Schlackenausscheidung rasch zu hohen Gewichtsabnahmen führen. Eine ausreichende Entsäuerung verhindert auch dauerhaft die erneute Einlagerung von Wasser und Schlacken und unterstützt den Fettabbau. Je nach Übersäuerung werden ein bis drei Rationen Basenpulver pro Tag empfohlen.

Als Begleitpräparat zur Entwässerung, Entsäuerung und Öffnung der Fettspeicher kann weiterhin ein basischer Tee Anwendung finden, beispielsweise das Produkt Herbaslim®. Der basische Tee liefert bei der Gewichtsabnahme wichtige Flüssigkeit zur Neutralisation und zum Abtransport der freigesetzten Schlacken und Gifte aus den Fettzellen. Ausreichendes Trinken (mind. 11 des basischen Tees plus viel Wasser ohne Kohlensäure) verhindert damit auch auf einfache Weise mögliche "Fastennebenwirkungen" wie Kopfschmerzen oder Mundgeruch. Der basische Tee unterstützt auch in Kombination mit dem Basenpulver, die Entsäuerung und damit den Abbau des Wasser-Schlackenübergewichts.

Der Mahlzeitenersatz in Form von Shakes beinhaltet:
- wenigstens ein Sojaeiweiss und/oder Milcheiweiss,
- wenigstens einen Mikrofaser-Balaststoff,
- wenigstens einen Kohlenhvdratlieferanten,
- wenigstens einen Fettsäurelieferanten,
- wenigstens ein fettlösliches und wenigstens ein wasserlösliches Vitamin,
- wenigstens einen Mineralstoff und
- wenigstens einen Geschmacksstoff.

Der Mahlzeitenersatz kann in einer bevorzugten Ausgestaltung weiterhin Isoflavone enthalten, wobei die Isoflavone besonders bevorzugt vorgesehen werden können, wenn der Mahlzeitenersatz Sojaeiweiss enthält.

Der Mahlzeitenersatz in Form eines oder mehrerer Shakes ist somit speziell für eine wirksame Gewichtsabnahme durch kalorienreduzierte Mahlzeiten mit Unterstützung der Thermogenese (Umwandlung von Kalorien in Wärme) aber auch für eine sichere und gesunde Gewichtsabnahme durch die Ausgewogenheit der wertvollen und lebenswichtigen Nähr- und Vitalstoffe konzipiert. Der Mahlzeitenersatz wird bevorzugt als Shake hergestellt.

Hochwertiges pflanzliches Sojaeiweiß mit einer sehr hohen biologischen Wertigkeit hilft die stoffwechselaktive und formgebende Muskulatur zu erhalten und damit die Gewichtsabnahme dauerhaft zu sichern. Durch den ausschließlichen Einsatz von Sojaeiweiß ist Der Mahlzeitenersatz in Form eines oder mehrerer Shakes ist zudem frei von Milcheiweiß. Weiterhin ist der Mahlzeitenersatz lactosefrei. Dadurch ist es auch für entsprechende Allergiker verträglich.

In einer alternativen Ausführung ist die Beimengung von Milcheiweiss vorgesehen, und zwar entweder zusätzlich zu Sojaeiweiss oder ausschließlich. Milcheiweiss stellt bekanntermaßen auch eine sehr hochwertige Eiweißquelle dar, insbesondere im Hinblick auf die Bildung von Muskulatur. Der Aufbau von Muskulatur ist sinnvoll, um in effektiver Weise Fett zu verbrennen.

Die Mikrofaser-Ballaststoffe bremsen den Heißhunger über einen verlangsamten Zuckereinstrom ins Blut und sorgen zudem für eine Entgiftung und eine bessere Verdauung. Die Kohlenhydrate garantieren Leistungsfähigkeit während der Gewichtsabnahme auch für das Gehirn und das Nervensystem. Darüber hinaus liefert das diätetische Lebensmittel lebensnotwendige Fettsäuren, Vitamine und Mineralstoffe. Mit Hilfe der Geschmacksstoffe werden verschiedene Geschmacksrichtungen erzeugt, so dass eine Abwechslung bei der Einnahme des Mahlzeitenersatzes entstehen kann und darüber hinaus für jede Person die richtige Geschmacksrichtung angeboten werden kann.

Die fakultativ beimengbaren und bei Shakes mit Sojaeiweiss vorzugsweise vorgesehenen Isoflavone sind sekundäre Pflanzenstoffe und wirken als Phytohormone (Phytoöstrogene). Durch den Zusatz von Isoflavonen können unter anderem Wechseljahrsbeschwerden verringert, gesunde stabile Knochen gefördert sowie Osteoporose und Brustkrebs vorgebeugt werden. Darüber hinaus können Isoflavone im Zusammenhang mit dem Cholesterinspiegel den LDL-Wert erniedrigen und den HDL-Wert erhöhen sowie das ProstataKrebs-Risiko beim Mann reduzieren. Isoflavone wirken Hormonspiegel normalisierend und dienen als Herz-Kreislauf-Schutz.

Der Mahlzeitenersatz in Form eines oder mehrerer Shakes greift speziell beim Fettübergewicht an, das längerfristig mobilisiert werden muss. Es wird empfohlen, ein bis zwei Mahlzeiten täglich durch den Mahlzeitenersatz in Form eines oder mehrerer Shakes zu ersetzen.

Insbesondere vorteilhaft ist es, wenn der Isoflavongehalt zwischen 10 mg/100 g Mahlzeitenersatz und 200 mg/100 g Mahlzeitenersatz, vorzugsweise zwischen 30 mg/100 g Mahlzeitenersatz und 60 mg/100 g Mahlzeitenersatz, insbesondere bei 33 mg/100 g Mahlzeitenersatz, liegt. Hinsichtlich weiterer Einzelheiten in Bezug auf den hier vorgeschlagenen Mahlzeitenersatz wird auf die EP 1 588 659 A2 der Anmelderin verwiesen.

Gemäß einer beispielhaften Ausführungsform des hier vorgeschlagenen Nahrungsergänzungsmittelpräparats kann die Wirksubstanzmischung wie folgt zusammengestellt sein:
Bockshornkleesamen-Konzentrat (Trigonella foenum-graecum L.): 1.413 mg
davon Ballaststoffe: 1.110 mg
Grüntee-Extrakt (Camelia Sinsensis): 492 mg
davon Polyphenole: 246 mg
davon Coffein: 39 mg
Orangenfrucht (Bitter-)Konzentrat (Citrus aurantium): 25 mg
Aroma (Curcumaextrakt): 20 mg
Aroma (ayurvedischer Aromaextrakt, Pfefferextrakt): 5 mg
Der Wirksubstanzmischung kann noch
MCT-Öl: 10 mg
beigemischt werden.

Das hier vorgeschlagene Nahrungsergänzungsmittelpräparat eignet sich insbesondere in Kombination mit einem Begleitpräparat zur Entwässerung sowie einem Mahlzeitenersatz in Form eines oder mehrerer Shakes, um Körper- bzw. Übergewicht, insbesondere Körperfett, auf verträgliche und nachhaltige Weise abzubauen.

## Patentansprüche

1. Nahrungsergänzungsmittelpräparat mit einer Wirksubstanzmischung, wobei die Wirksubstanzmischung enthält:
- Trigonella foenum-graecum L. (Bockshornkleesamen),
- Camelia sinsensis mit einem Anteil von Polyphenolen und Coffein,
- Curcumaextrakt,
- Ayurvedisches Gewürzextrakt, insbesondere Pfefferextrakt sowie
- Bitterorangenextrakt.

2. Nahrungsergänzungsmittelpräparat nach Anspruch 1,
**dadurch gekennzeichnet,dass**
Trigonella foenum-graecum L. in einem Anteil von 15 Gew.% bis 90 Gew.%, vorzugsweise in einem Anteil von etwa 65 Gew.% bis 80 Gew.%, in der Wirksubstanzmischung enthalten ist.

3. Nahrungsergänzungsmittelpräparat nach Anspruch 1 oder 2,
**dadurch gekennzeichnet,dass**
Camelia sinsensis mit einem Anteil von 5 Gew.% bis 50 Gew.%, vorzugsweise mit einem Anteil von 20 Gew.% bis 30 Gew.%, in der Wirksubstanzmischung enthalten ist.

4. Nahrungsergänzungsmittelpräparat nach einem der Ansprüche 1 bis 3,
**dadurch gekennzeichnet, dass**
Curcumaextrakt mit einem Anteil von 0,2 Gew.% bis 2,5 Gew.%, vorzugsweise mit einem Anteil von 0,7 Gew.% bis 1,3 Gew.%, in der Wirksubstanzmischung enthalten ist.

5. Nahrungsergänzungsmittelpräparat nach einem der Ansprüche 1 bis 4,
**dadurch gekennzeichnet, dass**
der ayurvedische Gewürzextrakt, insbesondere der Pfefferextrakt, mit einem Anteil von 0,05 Gew.% bis 0,8 Gew.%, vorzugsweise mit einem Anteil von 0,15 Gew.% bis 0,4 Gew.%, in der Wirksubstanzmischung enthalten ist.

6. Nahrungsergänzungsmittelpräparat nach einem der Ansprüche 1 bis 5,
**dadurch gekennzeichnet,dass**
Bitterorangenextrakt mit einem Anteil von 0,2 Gew.% bis 4,0 Gew.%, vorzugsweise mit einem Anteil von 0,6 Gew.% bis 2,0 Gew.%, in der Wirksubstanzmischung enthalten ist.

7. Nahrungsergänzungsmittelpräparat nach einem der Ansprüche 1 bis 6,
**dadurch gekennzeichnet, dass**
es insbesondere durch Bereitstellung in Kapseln so konfektioniert ist, dass die Tagesdosis für Trigonella foenum-graecum L. im Bereich von 300 mg bis 5 g, vorzugsweise im Bereich von 900 mg bis 1500 mg, und/oder die Tagesdosis von Camelia sinsensis im Bereich von 200 mg bis 2500 mg, vorzugsweise von 600 mg bis 1200 mg, und/oder die Tagesdosis von Curcumaextrakt im Bereich von 2 mg bis 100 mg, vorzugsweise im Bereich von 5 mg bis 30 mg, und/oder die Tagesdosis des ayurvedischen Gewürzextrakts, insbesondere des Pfefferextrakts, im Bereich von 1 mg bis 20 mg, vorzugsweise im Bereich von 2 mg bis 6 mg, und/oder die Tagesdosis des Bitterorangenextrakts im Bereich von 5 mg bis 50 mg, vorzugsweise im Bereich von 10 mg bis 30 mg, liegt.

8. Nahrungsergänzungsmittelpräparat nach einem der Ansprüche 1 bis 7,
**dadurch gekennzeichnet, dass**
der in Camelia Sinsensis enthaltene Anteil an Polyphenolen im Bereich von 5 Gew.% bis 20 Gew.%, vorzugsweise im Bereich von 8 Gew.% bis 15 Gew.%, an der Wirksubstanzmischung liegt und/oder eine Tagesdosis an Polyphenolen im Bereich von 75 mg bis 500 mg, vorzugsweise im Bereich von 150 mg bis 300 mg, vorgesehen ist.

9. Nahrungsergänzungsmittelpräparat nach einem der Ansprüche 1 bis 8,
**dadurch gekennzeichnet, dass**
der in Camelia Sinsensis enthaltene Anteil an Coffein im Bereich von 0,5 Gew.% bis 5 Gew.%, vorzugsweise in einem Bereich von 1,0 Gew.% bis 2,9 Gew.%, an der Wirksubstanz liegt, und/oder eine Tagesdosierung im Bereich von 10 mg bis 100 mg, vorzugsweise in einem Bereich von 20 mg bis 50 mg, vorgesehen ist.

10. Nahrungsergänzungsmittelpräparat nach einem der Ansprüche 1 bis 9,
**dadurch gekennzeichnet,dass**
die Wirksubstanz gegebenenfalls unter Beimengung von weiteren Träger- und Zusatzstoffen, insbesondere unter Beimengung von stoffwechselaktivem MCT-Öl, in oral einnehmbaren Kapseln konfektioniert wird.

11. Diätisches Lebensmittelset, umfassend:
- eine vorbestimmte Anzahl von Kapseln mit dem Nahrungsmittelpräparat nach einem der Ansprüche 1 bis 10,
- ein Begleitpräparat zur Entwässerung, Entsäuerung und Öffnung der Fettspeicher sowie
- einen Mahlzeitenersatz in Form eines oder mehrerer Shakes.

12. Diätisches Lebensmittelset nach Anspruch 11,
**dadurch gekennzeichnet, dass**
das Begleitpräparat die Mineralstoffe und Spurenelemente Kalzium, Magnesium, Selen, Kupfer, Zink, Chrom und Vitamin D enthält und dass das Begleitpräparat weiterhin mindestens einen Katalysator und/oder Wirkstoff, vorzugsweise in Pulverform, enthält, der die Resorption der Mineralstoffe und/oder Spurenelemente steigert und/oder verbessert und/oder beschleunigt.

13. Diätisches Lebensmittelset nach Anspruch 11 oder 12,
**dadurch gekennzeichnet, dass**
der Mahlzeitenersatz in Form von Shakes beinhaltet:
- wenigstens ein Sojaeiweiss und/oder ein Milcheiweiss,
- wenigstens einen Mikrofaser-Balaststoff,
- wenigstens einen Kohlenhydratlieferanten,
- wenigstens einen Fettsäurelieferanten,
- wenigstens ein fettlösliches und wenigstens ein wasserlösliches Vitamin,
- wenigstens einen Mineralstoff und
- wenigstens einen Geschmacksstoff.

14. Diätisches Lebensmittelset nach Anspruch 13,
**dadurch gekennzeichnet,dass**
der Mahlzeitenersatz weiterhin Isoflavone umfasst.

## Geänderte Patentansprüche

### Geänderte Patentansprüche gemäss Regel 137(2) EPÜ.

**1.** Nahrungsergänzungsmittelpräparat mit einer Wirksubstanzmischung, wobei die Wirksubstanzmischung enthält:
- Trigonella foenum-graecum L. (Bockshornkleesamen),
- Camelia sinsensis mit einem Anteil von Polyphenolen und Coffein,
- Curcumaextrakt,
- Pfefferextrakt sowie
- Bitterorangenextrakt.
